# EUROPEAN PATENT SPECIFICATION

(11) **EP 2 945 962 B1**
(45) Date of publication and mention of the grant of the patent: **17.04.2019**
(21) Application number: 14740523.7
(22) Date of filing: 17.01.2014
(51) Int. Cl.: C07K 1/14

(54) **METHODS FOR ISOLATING BLOOD PRODUCTS FROM AN INTER-ALPHA INHIBITOR PROTEIN-DEPLETED BLOOD PRODUCT MATERIAL**
VERFAHREN ZUR ISOLIERUNG VON BLUTPRODUKTEN AUS EINEM INTER-ALPHA-INHIBITORPROTEIN-ABGEREICHERTEN BLUTPRODUKTMATERIAL
PROCÉDÉS D'ISOLEMENT DE PRODUITS SANGUINS À PARTIR D'UNE MATIÈRE DE PRODUIT SANGUIN APPAUVRIE EN PROTÉINE INTER-ALPHA INHIBITEUR

(30) Priority: 18.01.2013 US 201361754366 P
(43) Date of publication of application: 25.11.2015
(73) Proprietor: Prothera Biologics, Inc., Providence, RI 02903 (US)
(72) Inventor: LIM, Yow-Pin, East Providence, RI 02903 (US)
(74) Representative: Helbig, Christian
(86) International application number: PCT/US2014/012033
(87) International publication number: WO 2014/113659

(56) References cited:
- WO-A2-2005/121163
- US-A1- 2003 190 732
- US-A1- 2011 190 194
- US-A1- 2011 293 594
- US-A1- 2012 053 113
- US-A1- 2012 053 113
- MCCANN K B ET AL: "Evaluation of expanded bed adsorption chromatography for extraction of prothrombin complex from Cohn Supernatant I", BIOLOGICALS, ACADEMIC PRESS LTD., LONDON, GB, vol. 36, no. 4, 1 July 2008 (2008-07-01), pages 227-233, XP022795225, ISSN: 1045-1056, DOI: 10.1016/J.BIOLOGICALS.2008.01.002 [retrieved on 2008-03-10]
- SALIER J-P ET AL: "The inter-alpha-inhibitor family: From structure to regulation", BIOCHEMICAL JOURNAL, PORTLAND PRESS LTD, GB, vol. 315, no. 1, 1 January 1996 (1996-01-01), pages 1-9, XP002241012, ISSN: 0264-6021

## Description

### BACKGROUND OF THE INVENTION

A variety of critical biological compounds are naturally present in blood. Commercially significant blood products include inter-alpha inhibitor proteins (IαIp), albumin, immunoglobulins (IVIg), factor VII, factor VIII, factor IX, alpha-1 antitrypsin, anti-thrombin III, C1-inhibitor, protein C, von Willebrand factor, factor H, prothrombin (factor II), and thrombin. Blood products serve key roles in clotting, immunity, inflammation, and other biological functions. Subjects deficient in one or more of these compounds may suffer from a variety of medical conditions; treatment with particular blood compounds may alleviate these conditions or their symptoms. Further, treatment with particular blood products can produce medical benefits, such as the prevention of sepsis or neuronal damage. The efficient purification of blood compounds is of particular importance in light of the limited supply of blood for this purpose.

Prior methods have focused on the isolation of IαIp from blood fractionation discard, but none focus on isolation from raw plasma, i.e., prior to the fractionation process.

### SUMMARY OF THE INVENTION

Isolation of multiple blood products from a single starting material maximizes the efficiency of blood product isolation. The present invention provides such a method. In the present invention, one or more blood products are isolated from a blood product material previously depleted of one or more inter-alpha inhibitor proteins (IαIp). This method provides new paths for increasing the efficiency of isolating blood components and providing pharmaceutically acceptable forms of those components, such as may be used by subjects in need.

The present invention provides a method for isolating one or more blood products from an inter-alpha inhibitor protein (IαIp)-depleted blood product material. In a first aspect, the method includes providing an IαIp-depleted blood product material that is depleted of one or more IαIp family members by at least about 20% of the total present in the source blood product material and that includes at least about 20% of IgG present in the source blood product material. The method further includes isolating one or more blood products from the IαIp-depleted blood product material, at least one of which is selected from albumin, IgA, IgG, IgM, IgD, IgG, IVIg, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, VII, factor VIII, factor IX, factor X, factor XI, factor XII, factor XIII, fibronectin, alpha-1 antitrypsin, alpha-2 antiplasmin, urokinase, C1-inhibitor, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II. In some embodiments, the IαIp-depleted blood product material substantially includes 3 or more non-lalp blood products, substantially includes 10 or more non-lalp blood products, or substantially includes 20 or more non-lalp blood products selected from albumin, IgA, IgG, IgM, IgD, IgG, IVIg, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-1 antitrypsin, alpha-2 antiplasmin, urokinase, C1-inhibitor, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II. In some embodiments, the isolating step includes the steps of contacting the IαIp-depleted blood product material to a support such that one or more of the blood products is substantially retained on the support, and subsequently eluting from the support a fraction enriched in at least one of the substantially retained blood products. In some embodiments, the support is a chromatography column. In any of the above embodiments, the IαIp-depleted blood product material may be a material depleted of one or more (e.g., two, three, four, or more) IαIp family members, such as IαI, PαI, and/or bikunin, or both IαI and PαI; the depletion of the one or more IαIp family members is by at least about 20% or by at least about 90% or more.

In another aspect of the invention, the method for isolating one or more blood products from an IαIp-depleted blood product material includes contacting to a first support a blood product material that includes at least IαIp, IgG in an IαIp family:IgG weight ratio equal to about 1:30, equal to about 1:5, or between about 1:30 and about 1:5, and one of factor VIII in a factor VIII:IαIp family weight ratio equal to or less than about 1:10⁶ and von Willebrand factor in a von Willebrand factor:IαIp family weight ratio equal to or less than about 1:40, by which IαIp is substantially retained on the first support and material not retained by the support is included in a first flow-through. This aspect further includes isolating at least one of the one or more blood products from the first flow-through, at least one of which is selected from albumin, IgA, IgG, IgM, IgD, IgG, IVIg, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, VII, factor VIII, factor IX, factor X, factor XI, factor XII, factor XIII, fibronectin, alpha-1 antitrypsin, alpha-2 antiplasmin, urokinase, C1-inhibitor, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II. In some embodiments, the blood product material is whole plasma, cryo-poor plasma, liquid plasma, fresh frozen plasma (FFP), FFP24, frozen plasma (FP), FP24, thawed FFP, thawed FFP24, thawed FP, thawed FP24, source plasma, recovered plasma, solvent/detergent-treated plasma (SDP), platelet-rich plasma (PRP), platelet-poor plasma (PPP), serum, blood, or a diluted or concentrated preparation thereof. In some embodiments, the blood product material may be admixed with loading buffer prior to contacting the first support. In certain embodiments, the loading buffer includes 10 to 300 mM salt, such as 10, 20, 30, 40, 50, 100, 150, 200, 250, or 300 mM salt. In certain embodiments, the loading buffer includes 50 to 250 mM salt, such as 50, 60, 70, 80, 90, 100, 150,200, or 250 mM salt.

In particular embodiments, the support is a QA support and the loading buffer includes about 10 to 100 mM salt, such as 10, 20, 30, 40, 50, 60, 75, 80, 85, 90, 95, or 100 mM salt. In some instances, the pH is from 5.5 to 6.5, such as 5.5, 5.7, 5.9, 6.0, 6.1, 6.3, or 6.5. In certain embodiments, the support is a QA support, the loading buffer includes about 50 mM salt, and the pH is about 6.0.

In particular embodiments, the support is a DEAE support and the loading buffer includes about 150 to 250 mM salt, such as 150, 175, 200, 225, or 250 mM salt. In some instances, the pH is from 7.0 to 8.0, such as 7.0, 7.2, 7.4, 7.6, 7.8, or 8.0. In certain embodiments, the support is a DEAE support, the loading buffer includes about 200 mM salt, and the pH is about 7.6.

In some embodiments, the first flow-through includes three or more non-lalp blood products, 10 or more non-lalp blood products, or 20 or more non-lalp blood products in an amount equal to or greater than about 20% of the amount present in the blood product material, and wherein each of said non-lalp blood products is selected from albumin, IgA, IgG, IgM, IgD, IgG, IVIg, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-1 antitrypsin, alpha-2 antiplasmin, urokinase, C1-inhibitor, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II.

In some embodiments, the isolating step includes contacting the first flow-through to a second support such that one or more of the blood products is substantially retained on the second support, and subsequently eluting from the second support a fraction enriched in at least one of the substantially retained blood products. In some embodiments, one or both of the first or second supports is a chromatography column. In some embodiments, the first support may be an anion exchange column. In certain embodiments, the first support may be a DEAE or QA column.

Further embodiments include eluting the substantially retained IαIp from the first support, producing a first eluate that is enriched with IαIp. The first eluate may consist of isolated IαIp. In some further embodiments, the method further includes isolating the substantially retained IαIp from the first eluate.

In some of the above embodiments, the yield of the isolated IαIp may be at least 5 µg/ml blood product material, at least 50 µg/ml blood product material, at least 100 µg/ml blood product material, at least 300 µg/ml blood product material, at least 600 µg/ml blood product material, or at least 900 µg/ml blood product material. In some of the above embodiments, the purity of isolated IαIp may be at least 5%, at least 25%, at least 50%, is at least 75%, or 100%. In some embodiments, the isolated IαIp may be IαI, PαI, or bikunin, or may include two or more IαIp family members, or may include IαI and PαI. In some embodiments, the substantially retained IαIp may be IαI, PαI, or bikunin, or may include two or more IαIp family members, or may include IαI and PαI.

In some embodiments, the yield of one or more isolated non-lalp blood products may be at least 20% of the total present in the first flow-through, at least 50% of the total present in the first flow-through, or at least 80% of the total present in the first flow-through. In some embodiments, the IαIp-depleted blood product material is a blood product material depleted of IαIp (e.g., one or more (e.g., two, three, four, or more) IαIp family members, such as IαI, PαI, and/or bikunin, or both IαI and PαI) by at least about 20% (e.g., by at least about 30%, 40%, 50%, 60%, 70%, 80%, 90%, 95%, or 99% or more, or a depletion within the range of 20%-90%) of the total IαIp present in the source blood product material. In some embodiments, the IαIp-depleted blood product material substantially includes 3 or more non-lalp blood products, substantially includes 10 or more non-lalp blood products, or substantially includes 20 or more non-lalp blood products selected from albumin, IgA, IgG, IgM, IgD, IgG, IVIg, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-1 antitrypsin, alpha-2 antiplasmin, urokinase, C1-inhibitor, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II.

In particular, the invention is directed to a method for isolating one or more blood products from an inter-alpha inhibitor protein (IαIp)-depleted blood product material, comprising:
(a) chromatographically depleting at least about 80% of IαIp from a blood product material selected from the group consisting of whole plasma, cryo-poor plasma, liquid plasma, fresh frozen plasma (FFP), FFP24, frozen plasma (FP), FP24, thawed FFP, thawed FFP24, thawed FP, thawed FP24, source plasma, recovered plasma, solvent/detergent-treated plasma (SDP), platelet-rich plasma (PRP), platelet-poor plasma (PPP), serum, blood, and a diluted or concentrated preparation thereof to produce the IαIp-depleted blood product material,
   wherein the IαIp-depleted blood product material comprises three or more blood products is selected from IVIg, alpha-1 antitrypsin, C1-inhibitor, albumin, IgA, IgG, IgM, IgD, IgG, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-2 antiplasmin, urokinase, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II; and
(b) chromatographically isolating one or more of the blood products from said IαIp-depleted blood product material by contacting said IαIp-depleted blood product material to a support such that one or more of said blood products is substantially retained on said support.

As used herein, the term "about" means +/- 10% of the recited value.

"Blood product" means a commercially valuable substance that may be naturally present in blood, for example in the blood of a human, and for which isolation from blood is commercially practiced. Blood products include IαIp, albumin, IgA, IgG, IgM, IgD, IgG, IVIg, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-1 antitrypsin, alpha-2 antiplasmin, urokinase, C1-inhibitor, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II.

"IαIp" means a blood product composed of one or more or all members of the inter-alpha inhibitor protein (IαIp) family, each member being composed of a light chain, also called bikunin, optionally linked to one or more heavy chains (e.g., heavy chains H1, H2, H3, and/or H4). Exemplary members of the IαIp family include inter-alpha inhibitor (IαI) composed of bikunin linked to 2 heavy polypeptide chains (e.g., both H1, both H2, or H1 and H2) and having a molecular weight of about 225 to about 260 kDA and pre-alpha inhibitor (PαI) composed of bikunin linked to a single heavy chain (e.g., H1, H2, H3, or H4) and having a molecular weight of about 110 to about 130 kDA. IαIp may be bikunin alone and/or the combination of bikunin with one or more heavy chains. "IαIp family" means all members of the IαIp family."Blood product material" means any blood-derived composition that includes at least IαIp and IgG in an IαIp family:IgG weight ratio equal to about 1:30, equal to about 1:5, or between about 1:30 and about 1:5, such as about 1:30, 1:25, 1:20, 1:15, 1:10, or 1:5, and one of factor VIII in a factor VIII:IαIp family weight ratio equal to or less than about 1:10⁶, such as about 1:10⁶, 1:10⁷, 1:10⁸, 1:10⁹, or 1:10¹⁰, and von Willebrand factor in a von Willebrand factor:IαIp family weight ratio equal to or less than about 1:40, such as about 1:40, 1:50, 1:60, 1:70, 1:80, 1:90, 1:100, or 1:200. A blood product material may be, for example, whole plasma, cryo-poor plasma, liquid plasma, thawed fresh frozen plasma (FFP), thawed FFP24, thawed frozen plasma (FP), thawed FP24, source plasma, recovered plasma, solvent/detergent-treated plasma (SDP), platelet-rich plasma (PRP), platelet-poor plasma (PPP), serum, blood, or a diluted or concentrated preparation thereof.

"Support" means any apparatus that interacts with at least one blood product in a manner that is dependent upon the properties of the blood product, such that the apparatus is useful in fractionating the blood products present in a mixture or solution. A support may be a column, a membrane, a disc, a chip, or other apparatus for chromatography or affinity capture, examples of which are known in the art.

"Substantially retained" means at least about 20%, such as about 20%, 30%, 40%, 50%, 60%, 70%, 80%, 90% or 100%, of the amount of a substance present in a starting material is captured on a support.

To "deplete" means to reduce the concentration or amount of a substance. The concentration or amount of a substance may be considered depleted if it is reduced by about 20% to about 100%, such as about 20%, 30%, 40%, 50%, 60%, 70%, 80%, 90%, or 100%.

"IαIp-depleted blood product material" means any material derived from a blood product material that is depleted of one or more or all IαIp family members and further includes IgG. More specifically, an IαIp-depleted blood product material includes no more than about 80%, such as about 80%, 70%, 60%, 50%, 40%, 30%, 20%, 10%, 1%, 0.1%, 0.01% or 0%, of the total of one or more or all lalp family members and at least about 20%, such as about 20%, 30%, 40%, 50%, 60%, 70%, 80%, 90% or 100%, of total IgG present in the starting blood product material. An IαIp-depleted blood product material may also include one or more additional blood products.

"Isolated" means to have separated about 20% to 100%, such as about 20%, 30%, 40%, 50%, 60%, 70%, 80%, 90% or 100%, of a blood product from a starting material. A starting material may be a blood product material, an IαIp-depleted blood product material, or an IαIp-depleted blood product material having been further depleted of one or more non-lalp blood products.

"Purity" means the extent to which a blood product that has been isolated, such as a blood product isolated by the methods of the present invention, is free of other components. Purity is expressed as the percentage by weight of blood product in an isolated blood product composition.

### BRIEF DESCRIPTION OF THE DRAWINGS

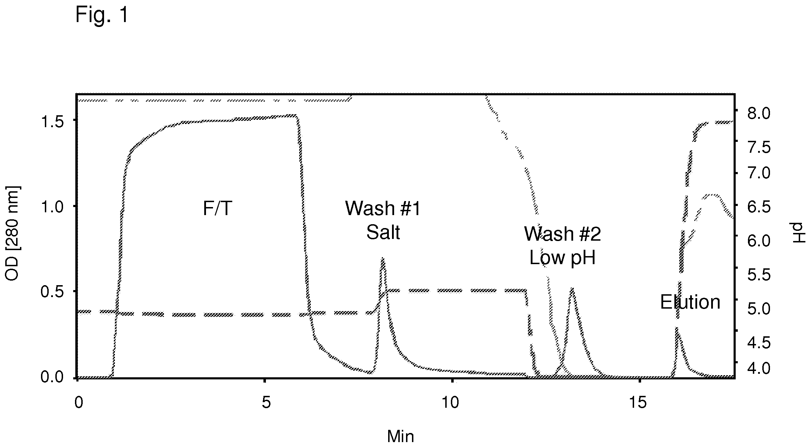

Fig. 1 is a chromatogram that shows the separation of cryo-poor plasma on a monolithic DEAE column (CIMMULTUS™). Cryo-poor plasma was diluted in 20 mM Tris buffer + 200 mM NaCl, pH 7.6 and loaded to an 8 mL DEAE column. After loading, unbound proteins were collected in the flow through fraction (F/T). The column was then washed with 290 mM NaCl (W#1) and 100 mM Acetate buffer, pH 2.95 (W#2). Inter-alpha inhibitor proteins were then eluted with a buffer containing 750 mM NaCl (EL). The F/T and wash fractions can be further processed to isolate other valuable therapeutic plasma proteins.

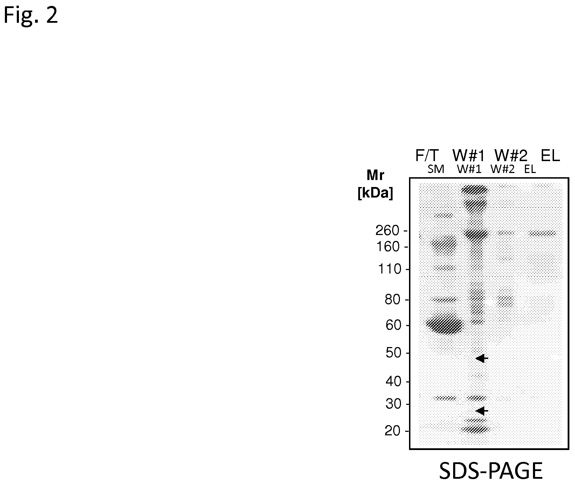

Fig. 2 is an SDS-PAGE analysis of the fractions produced by separation of cryo-poor plasma on a monolithic DEAE column. Cryo-poor plasma was diluted in 20 mM Tris buffer + 200 mM NaCl, pH 7.6 and loaded to an 8 mL DEAE column. After loading, unbound proteins were collected in the flow through fraction (F/T). The column was then washed with 290 mM NaCl (W#1) and 100 mM Acetate buffer, pH 2.95 (W#2). Inter-alpha inhibitor proteins were finally eluted with a buffer containing 750 mM NaCl (EL). The F/T and wash fractions can be further processed to isolate other valuable therapeutic plasma proteins.

### DETAILED DESCRIPTION

Blood product materials may contain numerous blood products, the isolation of some or all of which may be of medical or economic value. The present invention is directed toward a method of sequentially isolating multiple blood components from a single starting sample. More specifically, the discovery of the present invention is directed to methods of isolating one or more blood products from an IαIp-depleted blood product material.

The IαIp family is a group of structurally related plasma-associated serine protease inhibitors. Members of this family are composed of heavy and light polypeptide subunits that are covalently linked by a glycosaminoglycan. The light chain, also called bikunin, is responsible for the serine protease inhibitory activity of inter-alpha proteins. The name "bikunin" reflects the presence of two protease-inhibiting domains of the Kunitz type. The heavy chains of inter-alpha proteins (H1, H2, H3, H4) are also called Hyaluronic acid (HA) binding proteins. In normal plasma, bikunin is found mostly in a complex form as inter-alpha inhibitor (IαI), which has a molecular weight of about 225 to about 260 kDa, and pre-alpha inhibitor (PαI), which has molecular weight of about 110 to about 130 kDa. In IαI, bikunin is linked to 2 heavy polypeptide chains (e.g., H1 and H2), whereas, in PαI, only a single heavy chain (e.g., H3) is linked to bikunin.

The IαIp-depleted blood product material may be produced by the use of a support to isolate IαIp (e.g., one or more (e.g., two, three, four, or more, or all) IαIp family members, such as IαI, PαI, and/or bikunin, or both IαI and PαI) from a blood product material. In the present invention, IαIp may be isolated from a blood product material by any applicable method known in the art, including methods of chromatography, such as anion exchange chromatography, cation exchange chromatography, affinity chromatography, or dye-ligand chromatography. Exemplary methods for the isolation of IαIp may be found in US20110190194, which describes the use of DEAE chromatography with a low pH buffer, in particular a pH less than 4.0 (e.g., pH 4.0, 3.7, 3.5, 3.4, 3.3, 3.1, 2.9, 2.0), to isolate IαIp. The method may involve more than one buffer step, where the subsequent buffer may have a lower pH than the first. The buffer may be acetic acid, sodium acetate, citric acid, glycine, phosphate or salt buffer. For example, the first buffer may be a salt buffer with a salt concentration of 290 mM NaCl and the second buffer may have a pH of about 2.9. US20110190194 is herein incorporated by reference. Alternatively, US20120053113 describes the use of heparin affinity chromatography to isolate IαIp. Additional methods of isolating IαIp are known in the art. The IαIp-depleted blood product material may include the flow-through from a support capable of capturing IαIp, such as a chromatography column. If a wash is applied to this support, the wash may be optionally included in the IαIp-depleted blood product material or, alternatively, be itself an IαIp-depleted blood product material.

In some methods of the present invention, a blood product material may be admixed with a loading buffer before being applied to a support. A loading buffer of the present invention may be a buffer that permits or enhances the retention of one or more blood products on a support. A loading buffer of the present invention may also, or alternatively, decrease retention of contaminants on a support. A loading buffer may be a salt buffer of about 10 to about 300 mM salt, such as a salt buffer of about 10, 20, 30, 40, 50, 100, 120, 140, 160, 180, 200, 220, 240, 260, 280 or 300 mM salt.

In particular embodiments, the support is a QA support and the loading buffer includes about 10 to 100 mM salt, such as 10, 20, 30, 40, 50, 60, 75, 80, 85, 90, 95, or 100 mM salt. In some instances, the pH is from 5.5 to 6.5, such as 5.5, 5.7, 5.9, 6.0, 6.1, 6.3, or 6.5. In certain embodiments, the support is a QA support, the loading buffer includes about 50 mM salt, and the pH is about 6.0.

In particular embodiments, the support is a DEAE support and the loading buffer includes about 150 to 250 mM salt, such as 150, 175, 200, 225, or 250 mM salt. In some instances, the pH is from 7.0 to 8.0, such as 7.0, 7.2, 7.4, 7.6, 7.8, or 8.0. In certain embodiments, the support is a DEAE support, the loading buffer includes about 200 mM salt, and the pH is about 7.6.

Application of a blood product material admixed with a salt buffer may permit or enhance retention of IαIp while decreasing the retention of contaminants. Other examples of loading buffers are known in the art.

The IαIp-depleted blood product material may have in an IαIp family:IgG weight ratio less than about 1:30, such as about 1:40, 1:50, 1:100, 1:200, or 1:300, and one of factor VIII in a factor VIII:IαIp family weight ratio greater than about 1:10⁶, such as about 1:10⁵, 1:10⁴, 1:10³, 1:10², or 1:10, and von Willebrand factor in a von Willebrand factor:IαIp family weight ratio greater than about 1:40, such as 1:30, 1:20, 1:10, 1:5, or 1:1.

The present invention further relates to compositions required for or derived by the isolation of one or more blood products from an IαIp-depleted blood product material.

### Blood Products

Blood products of the present invention include IαIp, albumin, IgA, IgG, IgM, IgD, IgG, IVIg, anti-D IgG, hepatitis B IgG, measles IgG, rabies IgG, tetanus IgG, Varicella Zoster IgG, fibrinogen (factor I), prothrombin (factor II), factor III, factor V, VII, factor VIII, factor IX, factor X, factor XI, factor XII, factor XIII, fibronectin, alpha-1 antitrypsin, alpha-2 antiplasmin, urokinase, anti-thrombin III, C1-inhibitor, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, heparin cofactor II, and thrombin.

The inter-alpha inhibitor protein (IαIp) family is a group of plasma-associated serine protease inhibitors. Members of this family are composed of heavy chain and light chain (bikunin) polypeptide subunits that are covalently linked by a glycosaminoglycan. In these heavy and light chain forms, bikunin remains inactive until its release by partial proteolytic degradation, a mechanism that serves as a means to regulate activity. IαIp may inhibit serine proteases that are involved in inflammation, e.g., elastase, plasmin and cathepsin G. IαIp may be useful in the treatment of certain diseases and disorders, e.g., sepsis, septic shock, endotoxic shock, disseminated intravascular coagulation, and fibroproliferation.

Albumin is the main protein of plasma. The primary biological function of albumin is to regulate the colloidal osmotic pressure of blood. Albumin is capable of interacting with water, cations, fatty acids, hormones, bilirubin, thyroxine and other compounds. Albumin may be used to treat patients with blood loss, shock, severe burns or other medical conditions. It can also be used as a component of cell growth media or as an excipient for pharmacologically active compounds.

Immunoglobulins, or antibodies, are endogenous proteins which circulate in the blood and perform diverse functions. They are critical to immune function. Immunoglobulins are composed of four polypeptide chains, two light chains and two heavy chains. Immunoglobulin types are determined by the heavy chain, and include IgM, IgD, IgG, IgE and IgA. Immunoglobulins may be further defined by their specific compositions or functions.

Clotting factors are blood proteins that control bleeding by directing the clotting process. Circulating clotting factors are inactive, but injury initiates a coagulation cascade. The clotting process involves the contraction of blood vessels near a damaged area, followed by an accumulation of platelets. The platelets release chemical signals that result in the formation of a platelet plug. On the platelet surface, clotting factors form a fibrin clot. Clotting factors include factors I (fibrinogen), II (prothrombin), III (tissue factor), IV (calcium), V (labile factor), VII (stable factor), VIII (antihemophilic factor A), IX (antihemophilic factor B), X (Stuart Prower factor), XI (antihemophilic factor C), XII (Hageman factor), and XIII (fibrin stabilizing factor).

Fibrinogen is a plasma glycoprotein converted to fibrin by thrombin in the presence of calcium ions. Most of the fibrinogen found in blood is synthesized in the liver. During clotting, fibrin threads form a cross-linked meshwork that contributes to the formation of a blood clot. Levels of fibrinogen increase in association with inflammation, hemostatic stress, pregnancy and other medical conditions.

Tissue factor is a cell surface glycoprotein. Tissue factor interacts with stable factor, a serine protease, catalyzing the formation of thrombin from prothrombin. Some cells release tissue factor in response to blood vessel damage.

Antihemophilic factor A is a glycoprotein cofactor that circulates in complex with von Willebrand factor, from which it may be released by thrombin. Separately, Hageman factor, a serine protease, activates antihemophilic factor C, which in turn activates antihemophilic factor B. When thrombin dissociates antihemophilic factor A from von Willebrand factor, antihemophilic factor A can interact with antihemophilic factor B in the presence of calcium ions and phospholipids to form a complex that activates Stuart Prower factor, a vitamin K-dependent serine protease. Stuart Prower factor cleaves prothrombin to yield active thrombin, potentiating coagulation.

Fibrin stabilizing factor is the protein responsible for stabilizing the formation of a blood clot. Without it, blood clots form but break down, inhibiting wound healing. Fibrin stabilizing factor is a thrombin-activated transglutaminase that functions by forming amide cross links between fibrin molecules.

Alpha-1 antitrypsin is a protease inhibitor. Its concentration in blood may rise upon inflammation. It protects tissues from enzymes of inflammatory cells and inhibits a wide variety of proteases. For instance, it inhibits neutrophil elastase that would otherwise break down elastin and potentially result in respiratory complications such as emphysema or chronic obstructive pulmonary disease in adults or cirrhosis in children.

Anti-thrombin (III) is an inhibitor of the coagulation cascade. It is a protease that targets thrombin and factor X. Inhibitors of coagulation such as heparin act, in part, through the potentiation of anti-thrombin.

C1-inhibitor protein is a protease inhibitor that prevents spontaneous activation of the complement system. Its concentration in blood increases during inflammation. Targets may include C1r and C1s of the C1 complex of the complement pathway, MASP-1 and MASP-2 of the MBL complexes of the lectin pathway, kallikrein, FXI, FXII, and proteases of fibrinolytic, clotting, or kinin pathways. The activity of C1-inhibitor may indirectly prevent the cleavage of products such as C2, C4 and MBL. Protein C may also inhibit factors V and VIII.

Protein C is a zymogenic serine protease that may be activated by binding of thrombin. Upon activation, protein C may proteolytically inactivate factor V and factor VIII. Protein C contributes to the regulation of blood clotting, inflammation, and cell death. It also contributes to blood vessel permeability. Because of the important role protein C plays as an anticoagulant, protein C deficiency increases the risk of thrombosis.
von Willebrand factor is critical to blood clotting. It is a glue-like protein that interacts with platelets to form a plug that directs blood flow at or near an injury. If this factor is lacking or abnormal, a bleeding disorder may result.

Factor H regulates the alternative complement pathway. It possesses three heparin-binding sites. Mutation of factor H may result in atypical hemolytic uremic syndrome, a condition in which platelets are depleted.

Prothrombin is a trypsin-like serine protease glycoprotein with many functions. Proteolysis of prothrombin may generate thrombin.

Thrombin is a protease that cleaves Arg-Gly bonds of fibrinogen, resulting in the formation of fibrin and the release of fibrinopeptides A and B. Thrombin is part of the clotting cascade and contributes to the formation of a hemostatic plug. It potentiates coagulation by activating factors V, VIII, XI and XIII. Thrombin may also contribute to anticoagulation through interaction with thrombomodulin and activation of protein C. Thrombin may also contribute to inflammation and wound healing activities, for instance by activation of neutrophils or platelets.

### Methods of Isolating Blood Products

The present application describes methods for isolating one or more blood products from an IαIp-depleted blood product material. The starting blood product material for the purification of blood products may be, for example, whole plasma, cryo-poor plasma, liquid plasma, fresh frozen plasma (FFP), FFP24, frozen plasma (FP), FP24, thawed FFP, thawed FFP24, thawed FP, thawed FP24, source plasma, recovered plasma, solvent/detergent-treated plasma (SDP), platelet-rich plasma (PRP), platelet-poor plasma (PPP), serum, blood, or a diluted or concentrated preparation thereof.

In a first step of some methods of the present invention, a blood product material may be contacted to a first support capable of retaining IαIp. For instance, a blood product material may be contacted to a DEAE chromatography column, as described in US20110190194, or to a heparin affinity chromatography column, as described in US20120053113. The support of the present methods may be a support for a use in a method of chromatography, such as anion exchange chromatography, cation exchange chromatography, affinity chromatography, immunoaffinity chromatography, immobilized heparin chromatography, or dye-ligand chromatography. Examples of chromatography devices that may be used in the methods of the present invention include DEAE columns, such as DEAE SEPHAROSE® (GE Healthcare), DEAE Ceramic HYPERD® (Pall, e.g., 20067-C001), and FRACTOGEL® EMD DEAE (Merck Millipore, 1.16888). Further examples include Heparin affinity, such as Heparin SEPHAROSE® (GE Healthcare), Heparin Hyper D (Pall, e.g., 20029-021), and TSKGEL® Heparin (Tosoh, e.g., 14444). Alternatively, the support may be a support appropriate for nanofiltration. In some methods of the present invention, the support significantly retains the IαIp present in the blood product material.

In a second step, a first flow-through is collected from the first support. The first flow-through is an IαIp-depleted blood product material substantially inclusive of one or more non-lalp blood products. If the support is subsequently washed, the wash buffer flow-through may be included in the IαIp-depleted blood product or be itself an IαIp-depleted blood product. An IαIp-depleted blood product may comprise three or more, a majority, substantially all, or all of the non-lalp blood product that were present in the starting blood product material.

In some methods, IαIp may be eluted from the first support, producing a first eluate enriched with IαIp. This first eluate may also include a component of non-lalp blood products. A variety of methods known in the art may be applied to further isolate IαIp.

The percentage yield of IαIp that is isolated from a blood product material may be at least about 20%, such as about 20%, 30%, 40%, 50%, 60%, 70%, 80%, 90%, 95%, 99%, or 100%, of the total present in the blood product material.

The yield of IαIp from a blood product material may be at least about 5 µg/ml, such as about 5 µg/ml, 10 µg/ml, 20 µg/ml, 30 µg/ml, 40 µg/ml, 50 µg/ml, 100 µg/ml, 200 µg/ml, 300 µg/ml, 400 µg/ml, 500 µg/ml, 1000 µg/ml, or 1500 µg/ml.

In some alternative methods, the IαIp-depleted blood product is provided.

In the present invention, at least one non-lalp blood product is isolated from the IαIp-depleted blood product material. Isolation of a non-lalp blood product may involve applying the IαIp-depleted blood product material to a second support that captures one or more non-lalp blood products, and subsequently eluting those products from the second support. Methods for isolating non-lalp blood products are known in the art. Exemplary methods are provided below.

### Methods of Isolating Albumin

Albumin may be isolated from an IαIp-depleted blood product material by any applicable method known in the art, including precipitation, filtration, chromatography, liquid-solid extraction, and absorbance, or combinations thereof. For example, US2011137283 describes the purification of albumin by diafiltration, US4156681 describes the purification of albumin by alcohol extraction, and US4043997 describes the purification of albumin by selective absorbance with a polyhydroxy polymer. In further examples, US4177188 describes the purification of albumin by polyethylene glycol precipitation followed by thermocoagulation and US4086222 describes the purification of albumin by chromatography. Additional methods are also known in the art.

### Methods of Isolating Immunoglobulins

Immunoglobulins may be isolated from an IαIp-depleted blood product material by any applicable method known in the art, including precipitation, filtration, chromatography, liquid-solid extraction, and absorbance, or combinations thereof. For example, USRE31268 describes the purification of immunoglobulins by fractionated precipitation, US20120053325 describes the purification of immunoglobulins by dye-ligand affinity chromatography, and US4623541 describes the purification of immunoglobulins by a two-step ammonium sulfate fractionation procedure employing centrifugation and ion depletion. In some methods known in the art, individual immunoglobulin types are isolated by techniques with specificity for one or more of IgM, IgD, IgG, IgE and IgA, or particular immunoglobulins thereof. Additional methods are also known in the art.

### Methods of Isolating Factor I (Fibrinogen)

Factor I (fibrinogen) may be isolated from an IαIp-depleted blood product material by any applicable method known in the art, including precipitation, filtration, chromatography, liquid-solid extraction, and absorbance, or combinations thereof. For example, US7041790 describes the isolation of fibrinogen by an immobilized fibrinogen binding moiety conjugated to an affinity ligand. Suzuki et al. (Thrombosis Research, 18: 707-715, 1980) describes the isolation of fibrinogen by affinity chromatography with adsorption to a ristocetin-agarose column. Fibrinogen may also be isolated by cryoprecipitation followed by chemical precipitation using ethanol or ammonium sulfate (Ismail, Purification of Fibrinogen from Human Plasma. Chemical & Biomolecular Engineering Theses, Dissertations, & Student Research. 2012). Additional methods are also known in the art.

### Methods of Isolating Factor II (Prothrombin)

Factor II (prothrombin) may be isolated from an IαIp-depleted blood product material by any applicable method known in the art, including precipitation, filtration, chromatography, liquid-solid extraction, and absorbance, or combinations thereof. For example, US5143838 describes the isolation of prothrombin by anion exchange and US20120122179 describes the isolation of prothrombin by a deoxyribonucleic aptamer. Additional methods are also known in the art.

### Methods of Isolating Factor V (labile factor)

Factor V may be isolated from an IαIp-depleted blood product material by any applicable method known in the art, including precipitation, filtration, chromatography, liquid-solid extraction, and absorbance, or combinations thereof. For example, Chiu et al. describes the isolation of factor V by polyethylene glycol precipitation followed by an immunoaffinity column (Chiu et al, J. Clin Invest. 72: 493-503, 1983) Esnouf and Jobin describe the isolation of factor V by adsorption to a phosphorylated cellulose column optionally followed by ultrafiltration (Esnouf and Jobin, Biochem. J. 102: 660-665, 1967). Additional methods are also known in the art.

### Methods of Isolating Factor VII (stable factor)

Factor VII may be isolated from an IαIp-depleted blood product material by any applicable method known in the art, including precipitation, filtration, chromatography, liquid-solid extraction, and absorbance, or combinations thereof. For example, Bajaj et al. describe the isolation of Factor VII by adsorption onto barium citrate, followed by ammonium sulfate fractionation, DEAE-SEPHADEX® chromatography and preparative polyacrylamide gel electrophoresis (Bajaj et al., J. Biol. Chem. 256: 253-259, 1981). Kisiel and Davie isolate factor VII by barium sulfate adsorption followed by elution DEAE-SEPHADEX® batchwise adsorption and elution, benzamide-agarose column chromatography, heparin-agarose column chromatography and preparative polyacrylamide gel disc electrophoresis (Kisiel and Davie, Biochem. 14: 4928-4934, 1975). US4637932 describes the isolation of factor VII using a divalent metal salt adsorbent followed by an anionic exchange resin. Broze and Majerus describe the isolation of factor VII by barium citrate adsorption and elution and ammonium sulfate fractionation followed by two steps of QAE-SEPHADEX® column chromatography, SEPHADEX® G-100 column chromatography, and gel filtration on a SEPHADEX® G-25 column (Broze and Majerus, J. Biol. Chem. 255: 1242-1247, 1980). Hedner and Kisiel describe the isolation of factor VII by DEAE-SEPHAROSE® chromatography, ultra filtration, dialysis, QAE-SEPHADEX® A-50 chromatography, ultrafiltration, dialysis, and preparative electrophoresis (Hedner and Kisiel, J Clin. Invest. 71: 1836-1841, 1983). Additional methods are also known in the art.

### Methods of Isolating Factor VIII (antihemophilic factor A)

Factor VIII may be isolated from an IαIp-depleted blood product material by any applicable method known in the art, including precipitation, filtration, chromatography, liquid-solid extraction, and absorbance, or combinations thereof. For example, US4758657 describes the isolation of factor VIII:C by adsorption onto a hydrophobic interaction matrix. US4798675 describes the isolation of Factor VIII:C by adsorption onto a phospholipid coated support structure that is predominantly phosphatidylserine. US4789733 describes the isolation of factor VIII by precipitation with a sulphated polysaccharide, especially heparin. US5288853 describes the isolation of factor VIII complex using a heparin-coupled chromatographic medium, with further purification by precipitation with glycine and NaCl. US6143179 describes the isolation of factor VIII by affinity-chromatography with immobilized cellular von-Willebrand factor or a derivative thereof. US5259951 describes the isolation of factor VIII by ion exchange column chromatography. EP0317279 and US4361509 describe the isolation of factor VIII by immunoaffinity. US4758657 describes the isolation of factor VIII:C using a hydrophobic interaction matrix. US5245014 describes the isolation of factor VIII by gel filtration chromatography under group separation conditions. Additional methods are also known in the art.

### Methods of Isolating Factor IX (antihemophilic factor B)

Factor IX may be isolated from an IαIp-depleted blood product material by any applicable method known in the art, including precipitation, filtration, chromatography, liquid-solid extraction, and absorbance, or combinations thereof. For example, US5457181 describes the isolation of factor IX by DEAE-SEPHADEX® chromatography followed successively by ion-exchange chromatography on DEAE-SEPHAROSE® and affinity chromatography on heparin-SEPHAROSE®. US5919909 describes the isolation of factor IX by a precipitation step, preferably using ammonium sulfate, leaving factor IX in the supernatant, from which it is further purified by chromatography. Factor IX may also be isolated by a monoclonal antibody column, as described in US6732716. Additional methods are also known in the art.

### Methods of Isolating Factor X (Stuart Prower factor)

Factor X may be isolated from an IαIp-depleted blood product material by any applicable method known in the art, including precipitation, filtration, chromatography, liquid-solid extraction, and absorbance, or combinations thereof. For example, US5378365 describes the isolation of factor X by repeated ion exchange chromatographic separations followed by adsorption chromatography on metal ions. Bajaj et al. describe a procedure for the purification of prothrombin, factor IX and factor X with the initial steps of adsorption onto and elution from barium citrate, ammonium sulfate fractionation and DEAE-SEPHADEX® chromatography followed by heparin-agarose chromatography carried out in a (sodium) citrate buffer of pH 7.5 (Bajaj et al. Prep. Biochem. 11(4):397-412, 1981). Additional methods are also known in the art.

### Methods of Isolating Factor XI (antihemophilic factor C)

Factor XI may be isolated from an IαIp-depleted blood product material by any applicable method known in the art, including precipitation, filtration, chromatography, liquid-solid extraction, and absorbance, or combinations thereof. For example, US20100062512 describes the isolation of factor XI by hydrophobic charge induction chromatography. US5252217 isolates factor XI by a filtration-adsorption step and a single step of chromatography on cation exchange resin. Additional methods are also known in the art.

### Methods of Isolating XII (Hageman factor)

Factor XII may be isolated from an IαIp-depleted blood product material by any applicable method known in the art, including precipitation, filtration, chromatography, liquid-solid extraction, and absorbance, or combinations thereof. For example, Takahashi and Saito describe the isolation of factor XII by monoclonal antibody-immunoaffinity column chromatography followed by gel filtration (Takahashi and Saito, J. Biochem. 103: 641-643, 1988). Robin and Colman describe the isolation of factor XII by ammonium sulfate fractionation, two zinc chelate SEPHAROSE® affinity chromatography steps and gel filtration (Robin and Colman, Thrombosis Res. 41: 89-98, 1986). Chan and Movat describe the isolation of factor XII by adsorption with aluminum hydroxide, precipitation with polyethylene glycol, anion exchange chromatography on QAE- and a final step of either gel filtration on SEPHADEX® G-100 or affinity chromatography on an immunoadsorbent column (Chan and Movat, Thrombosis Res. 8: 337-349, 1976). Additional methods are also known in the art.

### Methods of Isolating factor XIII (fibrin stabilizing factor)

Factor XIII may be isolated from an IαIp-depleted blood product material by any applicable method known in the art, including precipitation, filtration, chromatography, liquid-solid extraction, and absorbance, or combinations thereof. For example, US5047506 describes the isolation of factor XIII by affinity chromatography. US20080176789 describes the isolation of a factor XIII polypeptide by sequential anion exchange chromatography and hydrophobic interaction chromatography. US5688919 describes the isolation of factor XIII by immunoaffinity chromatography. US20080281080 describes the isolation of factor XIII by immobilized metal affinity chromatography with optional further fractionation by various chromatography methods. US5204447 describes the isolation of factor XIII by precipitation by adjusting the pH of a biological fluid to about pH 5.5 to 6.5 and recovering the precipitated factor XIII. Additional methods are also known in the art.

### Methods of Isolating Alpha-1 Antitrypsin

Alpha-1 antitrypsin may be isolated from an IαIp-depleted blood product material by any applicable method known in the art, including precipitation, filtration, chromatography, liquid-solid extraction, and absorbance, or combinations thereof. For example, US20090292114 describes the purification of alpha-1 antitrypsin by at least two metal chelate chromatography steps and CN101274956 describes the purification of alpha-1 antitrypsin by precipitation, gel chromatography, and ultrafiltration. Additional methods are also known in the art.

### Methods of Isolating Anti-Thrombin (III)

Anti-thrombin (III) may be isolated from an IαIp-depleted blood product material by any applicable method known in the art, including precipitation, filtration, chromatography, liquid-solid extraction, and absorbance, or combinations thereof. For example, US3842061 describes the purification of anti-thrombin (III) by adsorption onto a water-insoluble gel matrix comprised primarily of cross-linked sulfated carbohydrate and US4510084 describes the purification of anti-thrombin (III) by interaction with heparin or heparinoid followed by adsorption with an anion exchanger. Additional methods are also known in the art.

### Methods of Isolating C1-Inhibitor Protein

C1-inhibitor protein may be isolated from an IαIp-depleted blood product material by any applicable method known in the art, including precipitation, filtration, chromatography, liquid-solid extraction, and absorbance, or combinations thereof. For example, US5030578 describes the purification of C1-inhibitor protein by PEG fractionation, jacalin-agarose chromatography and hydrophobic interaction chromatography on phenyl-SEPHAROSE®. In another example, US07/815870 describes the isolation of C1-inhibitor protein by antibody capture. Additional methods are also known in the art.

### Methods of Isolating Von Willebrand Factor

Von Willebrand factor may be isolated from an IαIp-depleted blood product material by any applicable method known in the art, including precipitation, filtration, chromatography, liquid-solid extraction, and absorbance, or combinations thereof. For example, US5854403 describes the isolation of von Willebrand factor by quaternary amino anion exchange. US7939643 describes the isolation of von Willebrand factor by hydroxylapatite chromatography. Additional methods are also known in the art.

### Methods of Isolating Factor H

Factor H may be isolated from an IαIp-depleted blood product material by any applicable method known in the art, including precipitation, filtration, chromatography, liquid-solid extraction, and absorbance, or combinations thereof. For example, US20120053113 describes the purification of factor H by cryoprecipitation and anion exchange. Additional methods are also known in the art.

### Methods of Isolating Thrombin

Thrombin may be isolated from an IαIp-depleted blood product material by any applicable method known in the art, including precipitation, filtration, chromatography, liquid-solid extraction, and absorbance, or combinations thereof. For example, US4965203 describes the purification of thrombin with a DEAE agarose column. Alternatively, thrombin may be produced from prothrombin that has been isolated from an IαIp-depleted blood product material. For example, US5393666 and US5677162 describe the treatment of prothrombin with calcium ions to yield thrombin and US5151355 describes the treatment of prothrombin with thromboplastin in the presence of calcium, followed by filtration, an anion-exchange agarose column, and a cation-exchange agarose column to yield thrombin. In another example, US5432062 describes the treatment of prothrombin with proteases in the presence of a detergent or certain chaotropic substances to produce thrombin. Additional methods are also known in the art.

### Purity and Yield

Isolation of a blood product may yield at least about 10%, such as about 10%, 20%, 30%, 40%, 50%, 60%, 70%, 80%, 90%, or 100%, of the total amount of that blood product present in the starting blood product material. The yield of a particular blood product isolated from a blood product material will depend, in part, upon the quantity of that blood product present in the blood product starting material. In some instances, the yield of a blood product isolated from a blood product material may be at least about 1 pg/ml starting material, such as about 1 pg/ml, 5 pg/ml, 10 pg/ml, 20 pg/ml, 30 pg/ml, 40 pg/ml, 50 pg/ml, 60 pg/ml, 70 pg/ml, 80 pg/ml, 100 pg/ml, 200 pg/ml, 300 pg/ml, 400 pg/ml, 500 pg/ml, or 1 ng/ml. In some instances, the yield of a blood product isolated from a blood product material may be at least about 5 ng/ml starting material, such as about 10 ng/ml, 20 ng/ml, 30 ng/ml, 40 ng/ml, 50 ng/ml, 60 ng/ml, 70 ng/ml, 80 ng/ml, 100 ng/ml, 200 ng/ml, 300 ng/ml, 400 ng/ml, 500 ng/ml, or 1 µg/ml. In some instances, the yield of a blood product isolated from a blood product material may be at least about 5 µg/ml starting material, such as about 10 µg/ml, 20 µg/ml, 30 µg/ml, 40 µg/ml, 50 µg/ml, 60 µg/ml, 70 µg/ml, 80 µg/ml, 90 µg/ml, 100 µg/ml, 200 µg/ml, 300 µg/ml, 400 µg/ml, or 500 µg/ml, 1 mg/ml, 2mg/ml, 3 mg/ml, 4 mg/ml, 5 mg/ml, 6 mg/ml, 7 mg/ml, 8 mg/ml, 9 mg/ml, 10 mg/ml, 20 mg/ml, 30 mg/ml, 40 mg/ml, 50 mg/ml, 60 mg/ml, 70mg/ml, 80 mg/ml, 90 mg/ml, 100 mg/ml, 200 mg/ml, 500 mg/ml, or 1 g/ml starting material. The yield of a blood product isolated from a blood product material may also be in the range of at least about 1 pg/ml to at least about 1 g/ml.

### Compositions

In addition to methods of isolating one or more blood products from an IαIp-depleted blood product material, the invention also features compositions that may be produced through the described methods. One of these compositions is an IαIp-depleted blood product material.

Isolated blood products are known to treat particular medical conditions. Blood products isolated by the methods of the present invention, including IαIp, may be useful in the treatment of such conditions. Pharmaceutical compositions of blood products isolated by the methods of the present invention may be administered or provided to a subject in need in a pharmaceutically acceptable dosage.

### EXAMPLE 1

In one example of the present invention, Von Willebrand factor is isolated from IαIp-depleted FFP24. FFP24 is thawed and diluted 1:10 in plasma dilution buffer (25 mM Tris, 200 mM NaCl, pH 7.6) and applied to a DEAE monolithic column. The flow-through is collected and additional plasma dilution buffer is applied to allow the starting material to pass through the column completely. The additional plasma dilution buffer may then be included with the flow-through. When the flow-through peak returns to baseline, the column is washed with low pH buffer (150 mM Acetic acid, pH 4.0, or 200 mM Acetic acid, pH 3.3) and the peak is collected. After the low pH wash, the column is further washed with a higher pH buffer (100 mM Tris, 100 mM NaCl, pH 7.6) to restore the pH. Bound protein is eluted with a high salt elution buffer (25 mM Tris, 1000 mM NaCl, pH 7.6). The peak is collected and this fraction contains highly pure IαIp. IαIp may then be further purified to exchange buffer and remove low molecular weight solutes and salts by ultrafiltration or diafiltration using a membrane cut off of 30 kDa.

Von Willebrand factor is isolated from the flow-through. The flow-through is filtered over an anion exchanger column (EMD-TMAE-FRACTOGEL® (Merck)) that has been equilibrated with buffer (20 mM Tris-HCI, pH 7.4). Subsequently, the column is washed with additional buffer. Foreign materials are removed by washing the column with 200 mM NaCl buffer. The Von Willebrand factor is then eluted from the column with another buffer (280 mM NaCl 20 mM Tris-HCI, pH 7.4). Subsequently, residual material, which is possibly present, is eluted from the column with 1M NaCl.

### EXAMPLE 2

In a second example of the present invention, albumin is isolated from IαIp-depleted cryo-poor plasma. Cryo-poor plasma is diluted 1:10 in dilution buffer (40 mM Tris, 200 mM NaCl, pH 7.6) and applied to a DEAE monolithic column. The flow-through is collected and additional buffer (25 mM Tris, 200 mM NaCl, pH 7.6) is applied to the column to allow the starting material to pass through the column completely. The additional buffer may then be included with the first flow-through. When the flow-through peak returns to baseline, the column is washed with salt-containing wash buffer (40 mM Tris-HCI, 290 mM NaCl, pH 7.6) and the peak is collected. After the salt wash, the column is additionally washed with low pH buffer (200 mM Na-Acetate, pH 2.95) and the peak is collected. Following the second wash, bound protein is eluted with high salt elution buffer (40 mM Na-Citrate, 1000 mM NaCl, pH 6.50). The peak is collected; this fraction contains highly pure IαIp.

Albumin is isolated from the flow-through. The flow-through is applied to a DEAE-SEPHADEX® A-50 (DEAE-substituted cross-linked dextran) that has been allowed to swell in 0.075M NaCl solution and has been decanted 3 times, autoclaved at 121 °C for 0.5 hours, washed with 1M NaCl, and suspended in 0.075M NaCl. The suspension is stirred for 45 minutes and then the DEAE-SEPHADEX® A-50 gel is filtered off, whereas the filtrate is frozen and stored at -20°C. This frozen suspension is thawed at +4°C and adjusted to pH 8.0 with 0.5M NaOH solution, after which polyethylene glycol 4000 (MW 3000 - 3700) is added to the pH adjusted plasma fraction. After stirring for 30 minutes at +4°C, the precipitate is removed by centrifugation at 1800 g for 10 minutes at +4°C. The supernatant is adjusted to pH 4.8 with 0.5M HCl at +4°C, and additional polyethylene glycol 4000 is added to a final concentration of 22% (w/v). The mixture is stirred at +4°C for 30 minutes and the albumin containing precipitate is collected by centrifugation at 1800 g for 10 minutes at +4°C. The precipitate is dissolved at +4°C in distilled water and pH is adjusted to 7.0 with 0.5M NaOH. The solution contains the albumin. Further purification of albumin is optionally achieved by application to an anion exchanger followed by application to a cation exchanger.

### EXAMPLE 3

In a third example of the present invention, Alpha-1 antitrypsin is isolated from IαIp-depleted whole plasma. Whole plasma is diluted 1:10 in dilution buffer (40 mM Tris, 200 mM NaCl, pH 7.6) and applied to a DEAE monolithic column. The flow-through is collected and additional buffer (25 mM Tris, 200 mM NaCl, pH 7.6) is applied to the column to allow the starting material to pass through the column completely. The additional buffer may then be included with the first flow-through. When the flow-through peak returns to baseline, the column is washed with salt-containing wash buffer (40 mM Tris-HCI, 290 mM NaCl, pH 7.6) and the peak is collected. After the salt wash, the column is additionally washed with low pH buffer (200 mM Na-Acetate, pH 2.95) and the peak is collected. Following the second wash, bound protein is eluted with high salt elution buffer (40 mM Na-Citrate, 1000 mM NaCl, pH 6.50). The peak is collected; this fraction contained highly pure IαIp.

To isolate Alpha-1 antitrypsin from the flow-through, the flow-through is frozen and subjected to a controlled thaw at -0.5°C to 2°C during which some proteins precipitate. The supernatant is collected, treated with CELITE®, and then filtered to remove unwanted proteins. The resulting supernatant is adjusted to a pH of 5.85 with acetate buffer and ethanol is added to 17-21% v/v. The temperature of the ensuing precipitation is maintained between -4°C and -6°C, such that the precipitate includes Fraction 1 and precipitate A of the Kistler and Nitschmann process (ibid). The supernatant is diluted 1:1 with buffer (10 mM NaH₂PO₄, 10 mM NaOH, pH 11). The pH of the resulting solution is between 6 and 7 with conductivity less than 7 mS/cm. pH is reduced to between 5.5 and 6.5 with dilute acetic acid just prior to loading onto a Capto Q SEPHAROSE® column equilibrated with buffer (20 mM phosphate, 30 mM NaCl, pH 6.2). Alpha-1 antitrypsin is then eluted with buffer (20 mM phosphate containing 170 mM NaCl, pH 6.2). 2.5 mM of imidazole is added to the Alpha-1 antitrypsin fraction eluted from the Capto Q column, which is then loaded onto a HisTrap column stripped of its nickel ions and re-charged with divalent copper cations. At this imidazole concentration and using this type of chelating solid support, some contaminants, but not Alpha-1 antitrypsin, bind to the solid support. The flow-through thus contains Alpha-1 antitrypsin.

To reduce the viral load of the Alpha-1 antitrypsin fraction, a polysorbate 20/tri-n-butyl phosphate mixture is added according to EP-A 0131740. The solvent detergent (SD) treated Alpha-1 antitrypsin fraction is loaded onto a chelating SEPHAROSE® solid support (iminodiacetic acid chelating ligand) charged with copper. Under the conditions of the load (2.5 mM imidazole in 20 mM phosphate buffer containing 30 mM NaCl, pH 6.2) the Alpha-1 antitrypsin is bound by the solid support whilst contaminants are not. The Alpha-1 antitrypsin is then eluted with 10 mM imidazole solution.

### OTHER EMBODIMENTS

While the invention has been described in connection with the specific embodiments, it will be understood that it is capable of further modifications. Therefore, this application is intended to cover any variations, uses, or adaptations of the invention that follow, in general, the principles of the invention, including departures from the present disclosure that come within known or customary practice within the art.

## Claims

1. A method for isolating one or more blood products from an inter-alpha inhibitor protein (IαIp)-depleted blood product material, comprising:
(a) chromatographically depleting at least about 80% of IαIp from a blood product material selected from the group consisting of whole plasma, cryo-poor plasma, liquid plasma, fresh frozen plasma (FFP), FFP24, frozen plasma (FP), FP24, thawed FFP, thawed FFP24, thawed FP, thawed FP24, source plasma, recovered plasma, solvent/detergent-treated plasma (SDP), platelet-rich plasma (PRP), platelet-poor plasma (PPP), serum, blood, and a diluted or concentrated preparation thereof to produce the IαIp-depleted blood product material,
wherein the IαIp-depleted blood product material comprises three or more blood products is selected from IVIg, alpha-1 antitrypsin, C1-inhibitor, albumin, IgA, IgG, IgM, IgD, IgG, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-2 antiplasmin, urokinase, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II; and
(b) chromatographically isolating one or more of the blood products from said IαIp-depleted blood product material by contacting said IαIp-depleted blood product material to a support such that one or more of said blood products is substantially retained on said support.

2. The method of claim 1, wherein said IαIp-depleted blood product material substantially comprises 10 or more, or 20 or more, non-lalp blood products selected from IVIg, alpha-1 antitrypsin, C1-inhibitor, albumin, IgA, IgG, IgM, IgD, IgG, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-2 antiplasmin, urokinase, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II.

3. The method of claim 1 or 2, wherein said isolating step (b) comprises the steps of eluting from said support a fraction enriched in at least one of said substantially retained blood products, optionally wherein said support is a chromatography column, membrane, disc, or chip.

4. The method of any one of claims 1-3, wherein
(i) said IαIp-depleted blood product material is a blood product material depleted of IαI by at least about 80% of the total present in the source blood product material, optionally wherein said IαIp-depleted blood product material is depleted of IαI by at least about 90% of the total present in the source blood product material;
(ii) said IαIp-depleted blood product material is a blood product material depleted of PαI by at least about 80% of the total present in the source blood product material, optionally wherein said IαIp-depleted blood product material is depleted of PαI by at least about 90% of the total present in the source blood product material;
(iii) said IαIp-depleted blood product material is a blood product material depleted of bikunin by at least about 80% of the total present in the source blood product material, optionally wherein said IαIp-depleted blood product material is depleted of bikunin by at least about 90% of the total present in the source blood product material;
(iv) said IαIp-depleted blood product material is a blood product material depleted of two or more IαIp family members by at least about 80% of the total present in the source blood product material, optionally wherein said IαIp-depleted blood product material is depleted of two or more IαIp family members by at least about 90% of the total present in the source blood product material; or
(v) said IαIp-depleted blood product material is a blood product material depleted of IαI and PαI by at least about 80% of the total present in the source blood product material, optionally wherein said IαIp-depleted blood product material is depleted of IαI and PαI by at least about 90% of the total present in the source blood product material.

5. The method of any one of claims 1-4, wherein said blood product material comprises IαIp, IgG in an IαIp family:IgG weight ratio equal to about 1:30, equal to about 1:5, or between about 1:30 and about 1:5, and one of factor VIII in a factor VIII:IαIp family weight ratio equal to or less than about 1:10⁶ and von Willebrand factor in a von Willebrand factor:IαIp family weight ratio equal to or less than about 1:40.

6. The method of any one of claims 1-5, wherein said blood product material is admixed with loading buffer prior to step (a)
optionally wherein said loading buffer comprises about 10 to about 300 mM salt, optionally wherein said loading buffer comprises about 50 to about 250 mM salt, optionally wherein said loading buffer comprises about 200 mM salt.

7. The method of any one of claims 1-6, wherein said IαIp-depleted blood product material comprises three or more, optionally ten or twenty or more, non-IαIp blood products in an amount equal to or greater than about 20% of the amount of each of said non-IαIp blood products present in said blood product material, and wherein said non-IαIp blood products are selected from IVIg, alpha-1 antitrypsin, C1-inhibitor, albumin, IgA, IgG, IgM, IgD, IgG, anti-D IgG, hepatitis B IgG, measles IgG, rabies IgG, tetanus IgG, Varicella Zoster IgG, fibrinogen (factor I), prothrombin (factor II), thrombin, anti-thrombin III, factor III, factor V, factor VII, factor VIII, factor IX, factor X, factor XI, factor XII, factor XIII, fibronectin, alpha-2 antiplasmin, urokinase, protein C, protein S, protein Z, protein Z-related protease inhibitor, plasminogen, tissue plasminogen activator, plasminogen activator inhibitor-1, plasminogen activator inhibitor-2, von Willebrand factor, factor H, prekallikrein, high-molecular-weight kininogen, and heparin cofactor II.

8. The method of any one of claims 1-7, wherein step (a) or step (b) comprises contacting the blood product material or the IαIp-depleted blood product material, respectively, to a chromatography column;
optionally wherein step (a) comprises contacting the blood product material to an anion exchange column, such as a DEAE or QA column.

9. The method of any one of claims 1-8, wherein step (a) comprises contacting the blood product material to a first support, wherein at least about 80% of the IαIp from said blood product material is retained on said support, and eluting said retained IαIp from said first support, thereby producing a first eluate, wherein said first eluate is enriched with the retained IαIp.

10. The method of claim 9, wherein said first eluate consists of isolated IαIp.

11. The method of claim 9, further comprising separating said retained IαIp from said first eluate to produce an isolated IαIp; optionally wherein:
(a) the yield of the isolated IαIp is at least 5 µg/ml blood product material, such as at least 50, 100, 300, 600, or 900 µg/ml blood product material;
(b) the purity of the isolated IαIp is at least 5%, such as at least 25%, 50%, 75%, or 100%;
(c) said isolated IαIp is IαI, PαI, or bikunin;
(d) said isolated IαIp comprises two or more IαIp family members, such as IαI and PαI;
(e) said retained IαIp is IαI, PαI, or bikunin; and/or
(f) said retained IαIp comprises two or more IαIp family members, such as IαI and PαI.

12. The method of any one of claims 1-11, wherein the yield of said one or more non-IαIp blood products isolated in step (b) is at least 20%, optionally at least 50% or at least 80%, of the total of each of said one or more non-IαIp blood products present in said IαIp-depleted blood product material, respectively.

## Patentansprüche

1. Verfahren zur Isolierung eines oder mehrerer Blutprodukte aus einem Inter-alpha-Inhibitorprotein (IαIp)-abgereicherten Blutproduktmaterial, umfassend:
(a) das chromatographische Abreichern von wenigstens etwa 80% IαIp aus einem Blutproduktmaterial, das ausgewählt ist aus der Gruppe, bestehend aus Vollplasma, kryoarmem Plasma, flüssigem Plasma, gefrorenem Frischplasma (GFP), GFP24, gefrorenem Plasma (FP), FP24, aufgetautem GFP, aufgetautem GFP24, aufgetautem FP, aufgetautem FP24, Quellplasma, rückgewonnenem Plasma, Lösungsmittel/Detergens-behandeltem Plasma (SDP), plättchenreichem Plasma (PRP), plättchenarmem Plasma (PPP), Serum, Blut, und einem verdünnten oder konzentrierten Präparat davon, um das IαIp-abgereicherte Blutproduktmaterial herzustellen,
wobei das IαIp-abgereicherte Blutproduktmaterial drei oder mehr Blutprodukte umfasst, die ausgewählt sind unter IVIg, Alpha-1-Antitrypsin, C1-Inhibitor, Albumin, IgA, IgG, IgM, IgD, IgG, Anti-D-IgG, Hepatitis-B-IgG, Masern-IgG, Tollwut-IgG, Tetanus-IgG, Varicella-Zoster-IgG, Fibrinogen (Faktor I), Prothrombin (Faktor II), Thrombin, Antithrombin III, Faktor III, Faktor V, Faktor VII, Faktor VIII, Faktor IX,
Faktor X, Faktor XI, Faktor XII, Faktor XIII, Fibronektin, Alpha2-Antiplasmin, Urokinase, Protein C, Protein S, Protein Z, dem Protein Z-abhängigen Proteaseinhibitor, Plasminogen, Gewebe-Plasminogenaktivator, Plasminogenaktivator-Inhibitor-1, Plasminogenaktivator-Inhibitor-2, von-Willebrand-Faktor, Faktor H, Präkallikrein, hochmolekularem Kininogen und Heparin-Cofaktor II, und
(b) das chromatographische Isolieren eines oder mehrerer der Blutprodukte aus dem IαIp-abgereicherten Blutproduktmaterial durch Kontaktieren des IαIp-abgereicherten Blutproduktmaterials mit einem Träger, so dass eines oder mehrere der Blutprodukte im Wesentlichen auf dem Träger zurückbehalten wird.

2. Verfahren nach Anspruch 1, wobei das IαIp-abgereicherte Blutproduktmaterial im Wesentlichen 10 oder mehr oder 20 oder mehr Nicht-IαIp-Blutprodukte umfasst, die ausgewählt sind unter IVIg, Alpha-1-Antitrypsin, C1-Inhibitor, Albumin, IgA, IgG, IgM, IgD, IgG, Anti-D-IgG, Hepatitis-B-IgG, Masern-IgG, Tollwut-IgG, Tetanus-IgG, Varicella-Zoster-IgG, Fibrinogen (Faktor I), Prothrombin (Faktor II), Thrombin, Antithrombin III, Faktor III, Faktor V, Faktor VII, Faktor VIII, Faktor IX, Faktor X, Faktor XI, Faktor XII, Faktor XIII, Fibronektin, Alpha2-Antiplasmin, Urokinase, Protein C,
Protein S, Protein Z, dem Protein Z-abhängigen Proteaseinhibitor, Plasminogen, Gewebe-Plasminogenaktivator, Plasminogenaktivator-Inhibitor-1, Plasminogenaktivator-Inhibitor-2, von-Willebrand-Faktor, Faktor H, Präkallikrein, hochmolekularem Kininogen und Heparin-Cofaktor II.

3. Verfahren nach Anspruch 1 oder 2, wobei der Isolierschritt (b) die Schritte umfasst: Eluieren einer Fraktion aus dem Träger, die mit wenigstens einem der im Wesentlichen zurückbehaltenen Blutprodukte angereichert ist, gegebenenfalls wobei der Träger ein(e) Chromatographiesäule, Membran, Scheibe oder Chip ist.

4. Verfahren nach einem der Ansprüche 1 bis 3, wobei
(i) das IαIp-abgereicherte Blutproduktmaterial ein Blutproduktmaterial ist, das an IαI um wenigstens etwa 80% der in dem Quell-Blutproduktmaterial vorhandenen Gesamtmenge abgereichert ist, gegebenenfalls wobei das IαIp-abgereicherte Blutproduktmaterial an IαI um wenigstens etwa 90% der in dem Quell-Blutproduktmaterial vorhandenen Gesamtmenge abgereichert ist;
(ii) das IαIp-abgereicherte Blutproduktmaterial ein Blutproduktmaterial ist, das an PαI um wenigstens etwa 80% der in dem Quell-Blutproduktmaterial vorhandenen Gesamtmenge abgereichert ist, gegebenenfalls wobei das IαIp-abgereicherte Blutproduktmaterial an PαI um wenigstens etwa 90% der in dem Quell-Blutproduktmaterial vorhandenen Gesamtmenge abgereichert ist;
(iii) das IαIp-abgereicherte Blutproduktmaterial ein Blutproduktmaterial ist, das an Bikunin um wenigstens etwa 80% der in dem Quell-Blutproduktmaterial vorhandenen Gesamtmenge abgereichert ist, gegebenenfalls wobei das IαIp-abgereicherte Blutproduktmaterial an Bikunin um wenigstens etwa 90% der in dem Quell-Blutproduktmaterial vorhandenen Gesamtmenge abgereichert ist;
(iv) das IαIp-abgereicherte Blutproduktmaterial ein Blutproduktmaterial ist, das an zwei oder mehr IαIp-Familienmitgliedern um wenigstens etwa 80% der in dem Quell-Blutproduktmaterial vorhandenen Gesamtmenge abgereichert ist, gegebenenfalls wobei das IαIp-abgereicherte Blutproduktmaterial an zwei oder mehr IαIp-Familienmitgliedern um wenigstens etwa 90% der in dem Quell-Blutproduktmaterial vorhandenen Gesamtmenge abgereichert ist, oder
(v) das IαIp-abgereicherte Blutproduktmaterial ein Blutproduktmaterial ist, das an IαI und PαI um wenigstens etwa 80% der in dem Quell-Blutproduktmaterial vorhandenen Gesamtmenge abgereichert ist, gegebenenfalls wobei das lalp-abgereicherte Blutproduktmaterial an IαI und PαI um wenigstens etwa 90% der in dem Quell-Blutproduktmaterial vorhandenen Gesamtmenge abgereichert ist.

5. Verfahren nach einem der Ansprüche 1 bis 4, wobei das Blutproduktmaterial IαIp und IgG in einem Gewichtsverhältnis von IαIp-Familie-IgG von etwa 1:30, etwa 1:5 oder zwischen etwa 1:30 und etwa 1:5, und einen der Faktoren Faktor VIII in einem Gewichtsverhältnis von Faktor VIII:IαIp-Familie kleiner oder gleich etwa 1:10⁶ und von-Willebrand-Faktor in einem Gewichtsverhältnis von von-Willebrand-Faktor-IαIp-Familie kleiner oder gleich etwa 1:40 umfasst.

6. Verfahren nach einem der Ansprüche 1 bis 5, wobei das Blutproduktmaterial vor dem Schritt (a) mit Ladepuffer vermischt wird,
gegebenenfalls wobei der Ladepuffer etwa 10 bis etwa 300 mM Salz umfasst, gegebenenfalls wobei der Ladepuffer etwa 50 bis etwa 250 mM Salz umfasst, gegebenenfalls wobei der Ladepuffer etwa 200 mM Salz umfasst.

7. Verfahren nach einem der Ansprüche 1 bis 6, wobei das IαIp-abgereicherte Blutproduktmaterial drei oder mehr, gegebenenfalls zehn oder zwanzig oder mehr Nicht-IαIp-Blutprodukte in einer Menge größer oder gleich etwa 20% der Menge jedes der in dem Blutproduktmaterial vorhandenen Nicht-IαIp-Blutprodukte umfasst, und wobei die Nicht-IαIp-Blutprodukte ausgewählt sind unter IVIg, Alpha-1-Antitrypsin, C1-Inhibitor, Albumin, IgA, IgG, IgM, IgD, IgG, Anti-D-IgG, Hepatitis-B-IgG, Masern-IgG, Tollwut-IgG, Tetanus-IgG, Varicella-Zoster-IgG, Fibrinogen (Faktor I), Prothrombin (Faktor II), Thrombin, Antithrombin III, Faktor III, Faktor V, Faktor VII, Faktor VIII, Faktor IX, Faktor X, Faktor XI, Faktor XII, Faktor XIII, Fibronektin, Alpha2-Antiplasmin, Urokinase, Protein C, Protein S, Protein Z, dem Protein Z-abhängigen Proteaseinhibitor, Plasminogen, Gewebe-Plasminogenaktivator, Plasminogenaktivator-Inhibitor-1, Plasminogenaktivator-Inhibitor-2, von-Willebrand-Faktor, Faktor H, Präkallikrein, hochmolekularem Kininogen und Heparin-Cofaktor II.

8. Verfahren nach einem der Ansprüche 1 bis 7, wobei Schritt (a) oder Schritt (b) das Kontaktieren des Blutproduktmaterials bzw. des IαIp-abgereicherten Blutproduktmaterials mit einer Chromatographiesäule umfasst;
gegebenenfalls wobei Schritt (a) das Kontaktieren des Blutproduktmaterials mit einer Anionenaustauschersäule, wie einer DEAE- oder QA-Säule, umfasst.

9. Verfahren nach einem der Ansprüche 1 bis 8, wobei Schritt (a) umfasst: das Kontaktieren des Blutproduktmaterials mit einem ersten Träger, wobei wenigstens etwa 80% des IαIp aus dem Blutproduktmaterial auf dem Träger zurückbehalten werden, und das Eluieren des zurückbehaltenen IαIp aus dem ersten Träger, wodurch ein erstes Eluat hergestellt wird, wobei das erste Eluat mit dem zurückbehaltenen IαIp angereichert ist.

10. Verfahren nach Anspruch 9, wobei das erste Eluat aus isoliertem IαIp besteht.

11. Verfahren nach Anspruch 9, ferner umfassend das Abtrennen des zurückbehaltenen IαIp aus dem ersten Eluat, um ein isoliertes IαIp herzustellen, gegebenenfalls wobei:
(a) die Ausbeute an isoliertem IαIp wenigstens 5 µg/ml Blutproduktmaterial, insbesondere wenigstens 50, 100, 300, 600 oder 900 µg/ml Blutproduktmaterial, beträgt;
(b) die Reinheit des isolierten IαIp wenigstens 5%, insbesondere wenigstens 25%, 50%, 75% oder 100%, beträgt;
(c) das isolierte IαIp IαI, PαI oder Bikunin ist;
(d) das isolierte IαIp zwei oder mehr lalp-Familienmitglieder, insbesondere IαI und PαI, umfasst;
(e) das zurückbehaltene IαIp IαI, PαI oder Bikunin ist, und/oder
(f) das zurückbehaltene IαIp zwei oder mehr IαIp-Familienmitglieder, insbesondere IαI und PαI, umfasst.

12. Verfahren nach einem der Ansprüche 1 bis 11, wobei die Ausbeute des einen oder der mehreren in Schritt (b) isolierten Nicht-IαIp-Blutprodukte wenigstens 20%, gegebenenfalls wenigstens 50% oder wenigstens 80%, der in dem IαIpabgereicherten Blutproduktmaterial vorhandenen Gesamtmenge an Nicht-IαIp-Blutprodukten beträgt.

## Revendications

1. Procédé d'isolement d'un ou plusieurs produits sanguins à partir d'une matière de produit sanguin appauvrie en protéine inter-alpha inhibiteur (IαIp), ledit procédé comportant les étapes suivantes:
(a) appauvrissement chromatographique d'au moins 80% d'IαIp à partir d'une matière de produit sanguin choisie dans le groupe constitué par le plasma entier, le plasma pauvre en cryoprécipité, le plasma liquide, le plasma frais congelé (PFC), le PFC24, le plasma congelé (PC), le PC24, le PFC décongelé, le PFC24 décongelé, le PC décongelé, le PC24 décongelé, le plasma de source, le plasma récupéré, le plasma traité par solvant détergent (PSD), le plasma riche en plaquettes (PRP), le plasma pauvre en plaquettes (PPP), le sérum, le sang, et une préparation diluée ou concentrée de ceux-ci, afin de produire la matière de produit sanguin appauvrie en IαIp,
où la matière de produit sanguin appauvrie en IαIp comporte trois produits sanguins ou plus choisis parmi l'IVIg, l'alpha-1-antitrypsine, le C1-inhibiteur, l'albumine, l'IgA, l'IgG, l'IgM, l'IgD, l'IgG, l'IgG anti-D, l'IgG de l'hépatite B, l'IgG de la rougeole, l'IgG de la rage, l'IgG du tétanos, l'IgG antivaricelleuse-antizostérienne, le fibrinogène (facteur I), la prothrombine (facteur II), la thrombine, l'antithrombine III, le facteur III, le facteur V, le facteur VII, le facteur VIII, le facteur IX, le facteur X, le facteur XI, le facteur XII,
le facteur XIII, la fibronectine, l'alpha-2-antiplasmine, l'urokinase, la protéine C, la protéine S, la protéine Z, l'inhibiteur dépendant de la protéine Z, le plasminogène, l'activateur tissulaire du plasminogène, l'inhibiteur de l'activateur du plasminogène 1, l'inhibiteur de l'activateur du plasminogène 2, le facteur de von Willebrand, le facteur H, la prékallikréine, le kininogène à poids moléculaire élevé, et le cofacteur d'héparine II; et
(b) isolement chromatographique d'un ou plusieurs des produits sanguins à partir de ladite matière de produit sanguin appauvrie en IαIp par mise en contact de ladite matière de produit sanguin appauvrie en IαIp avec un support de sorte qu'un ou plusieurs desdits produits sanguins soit sensiblement retenu sur ledit support.

2. Procédé selon la revendication 1, dans lequel la matière de produit sanguin appauvrie en IαIp comporte sensiblement 10 ou plus, ou 20 ou plus, produits sanguins non-IαIp choisis parmi l'IVIg, l'alpha-1-antitrypsine, le C1-inhibiteur, l'albumine, l'IgA, l'IgG, l'IgM, l'IgD, l'IgG, l'IgG anti-D, l'IgG de l'hépatite B, l'IgG de la rougeole, l'IgG de la rage, l'IgG du tétanos, l'IgG antivaricelleuse-antizostérienne, le fibrinogène (facteur I), la prothrombine (facteur II), la thrombine, l'antithrombine III, le facteur III, le facteur V, le facteur VII, le facteur VIII, le facteur IX, le facteur X, le facteur XI, le facteur XII, le facteur XIII, la fibronectine, l'alpha-2-antiplasmine, l'urokinase, la protéine C, la protéine S, la protéine Z, l'inhibiteur dépendant de la protéine Z, le plasminogène, l'activateur tissulaire du plasminogène, l'inhibiteur de l'activateur du plasminogène 1, l'inhibiteur de l'activateur du plasminogène 2, le facteur de von Willebrand, le facteur H, la prékallikréine, le kininogène à poids moléculaire élevé, et le cofacteur d'héparine II.

3. Procédé selon la revendication 1 ou 2, dans lequel l'étape d'isolement (b) comporte les étapes d'élution, à partir dudit support, d'une fraction enrichie en au moins un des produits sanguins sensiblement retenus, optionnellement dans lequel ledit support est une colonne chromatographique, une membrane, un disque ou une puce.

4. Procédé selon l'une quelconque des revendications 1 à 3, dans lequel
(i) ladite matière de produit sanguin appauvrie en IαIp est une matière de produit sanguin appauvrie en IαI d'au moins environ 80% de la quantité totale présente dans la matière de produit sanguin de source, optionnellement dans lequel ladite matière de produit sanguin appauvrie en IαIp est appauvrie en IαI d'au moins environ 90% de la quantité totale présente dans la matière de produit sanguin de source;
(ii) ladite matière de produit sanguin appauvrie en IαIp est une matière de produit sanguin appauvrie en PαI d'au moins environ 80% de la quantité totale présente dans la matière de produit sanguin de source, optionnellement dans lequel ladite matière de produit sanguin appauvrie en IαIp est appauvrie en PαI d'au moins environ 90% de la quantité totale présente dans la matière de produit sanguin de source;
(iii) ladite matière de produit sanguin appauvrie en IαIp est une matière de produit sanguin appauvrie en bikunine d'au moins environ 80% de la quantité totale présente dans la matière de produit sanguin de source, optionnellement dans lequel ladite matière de produit sanguin appauvrie en IαIp est appauvrie en bikunine d'au moins environ 90% de la quantité totale présente dans la matière de produit sanguin de source;
(iv) ladite matière de produit sanguin appauvrie en IαIp est une matière de produit sanguin appauvrie en deux ou plus membres de la famille IαIp d'au moins environ 80% de la quantité totale présente dans la matière de produit sanguin de source, optionnellement dans lequel ladite matière de produit sanguin appauvrie en IαIp est appauvrie en deux ou plus membres de la famille IαIp d'au moins environ 90% de la quantité totale présente dans la matière de produit sanguin de source; ou
(v) ladite matière de produit sanguin appauvrie en IαIp est une matière de produit sanguin appauvrie en IαI et PαI d'au moins environ 80% de la quantité totale présente dans la matière de produit sanguin de source, optionnellement dans lequel ladite matière de produit sanguin appauvrie en IαIp est appauvrie en IαI et PαI d'au moins environ 90% de la quantité totale présente dans la matière de produit sanguin de source.

5. Procédé selon l'une quelconque des revendications 1 à 4, dans lequel la matière de produit sanguin comporte l'IαIp et l'IgG dans un rapport de poids de famille IαIp:IgG égal à environ 1:30, égal à environ 1:5, ou entre environ 1:30 et environ 1:5, et l'un des facteurs suivants: facteur VIII dans un rapport de poids de facteur VIII:famille IαIp égal ou inférieur à environ 1:10⁶ et facteur de von Willebrand dans un rapport de poids de facteur de von Willebrand:famille IαIp égal ou inférieur à environ 1:40.

6. Procédé selon l'une quelconque des revendications 1 à 5, dans lequel ladite matière de produit sanguin est mélangée avec un tampon de dissociation avant l'étape (a),
optionnellement dans lequel ledit tampon de dissociation comporte d'environ 10 à environ 300 mM de sel,
optionnellement dans lequel ledit tampon de dissociation comporte d'environ 50 à environ 250 mM de sel, optionnellement dans lequel ledit tampon de dissociation comporte environ 200 mM de sel.

7. Procédé selon l'une quelconque des revendications 1 à 6, dans lequel la matière de produit sanguin appauvrie en IαIp comporte trois produits sanguins non-IαIp ou plus, optionnellement dix ou vingt ou plus, dans une quantité égale ou supérieure à environ 20% de la quantité de chacun des produits sanguins non-IαIp présents dans la matière de produit sanguin, et dans lequel lesdits produits sanguins non-IαIp sont choisis parmi l'IVIg, l'alpha-1-antitrypsine, le C1-inhibiteur, l'albumine, l'IgA, l'IgG, l'IgM, l'IgD, l'IgG, l'IgG anti-D, l'IgG de l'hépatite B, l'IgG de la rougeole, l'IgG de la rage, l'IgG du tétanos, l'IgG antivaricelleuse-antizostérienne, le fibrinogène (facteur I), la prothrombine (facteur II), la thrombine, l'antithrombine III, le facteur III, le facteur V, le facteur VII, le facteur VIII, le facteur IX, le facteur X, le facteur XI, le facteur XII, le facteur XIII, la fibronectine, l'alpha-2-antiplasmine, l'urokinase, la protéine C, la protéine S, la protéine Z, l'inhibiteur dépendant de la protéine Z, le plasminogène, l'activateur tissulaire du plasminogène, l'inhibiteur de l'activateur du plasminogène 1, l'inhibiteur de l'activateur du plasminogène 2, le facteur de von Willebrand, le facteur H, la prékallikréine, le kininogène à poids moléculaire élevé, et le cofacteur d'héparine II.

8. Procédé selon l'une quelconque des revendications 1 à 7, dans lequel l'étape (a) ou l'étape (b) comporte la mise en contact de la matière de produit sanguin ou de la matière de produit sanguin appauvrie en IαIρ, respectivement, avec une colonne chromatographique;
optionnellement dans lequel l'étape (a) comporte la mise en contact de la matière de produit sanguin à une colonne échangeuse d'anions, telle qu'une colonne DEAE ou QA.

9. Procédé selon l'une quelconque des revendications 1 à 8, dans lequel l'étape (a) comporte la mise en contact de la matière de produit sanguin avec un premier support, au moins environ 80% de l'IαIp de ladite matière de produit sanguin étant retenu sur ledit support, et l'élution de l'IαIp retenu à partir dudit premier support, produisant ainsi un premier éluat, ledit premier éluat étant enrichi en l'IαIp retenu.

10. Procédé selon la revendication 9, dans lequel ledit éluat consiste d'IαIp isolé.

11. Procédé selon la revendication 9, comportant en outre la séparation de l'IαIp retenu dudit premier éluat afin de produire un IαIp isolé, optionnellement dans lequel:
(a) le rendement de l'IαIp isolé est d'au moins 5 µg/ml de matière de produit sanguin, notamment au moins 50, 100, 300, 600, ou 900 µg/ml de matière de produit sanguin;
(b) la pureté de l'IαIp isolé est d'au moins 5%, notamment au moins 25%, 50%, 75%, ou 100%;
(c) l'IαIp isolé est l'IαI, le PαI, ou la bikunine;
(d) l'IαIp isolé comporte deux ou plus membres de la famille IαIρ, notamment l'IαI et le PαI;
(c) l'IαIρ retenu est l'IαI, le PαI, ou la bikunine; et/ou
(f) l'IαIp retenu comporte deux ou plus membres de la famille IαIρ, notamment l'IαI et le PαI.

12. Procédé selon l'une quelconque des revendications 1 à 11, dans lequel le rendement dudit un ou desdits plusieurs produits sanguins non-IαIp isolés à l'étape (b) est d'au moins 20%, optionnellement d'au moins 50% ou d'au moins 80%, de la quantité totale de chacun des un ou plusieurs produits sanguins non-IαIp présents dans ladite matière de produit sanguin appauvrie en IαIp, respectivement.
